# EUROPÄISCHE PATENTSCHRIFT

(11) **EP 1 844 106 B1**
(45) Veröffentlichungstag und Bekanntmachung des Hinweises auf die Patenterteilung: **11.07.2012**
(21) Anmeldenummer: 06706222.4
(22) Anmeldetag: 13.01.2006
(51) Int. Cl.: C08L 83/08

(54) **GUANIDINOGRUPPEN- HALTIGE SILOXANE UND DEREN VERWENDUNG FÜR KOSMETISCHE FORMULIERUNGEN**
SILOXANES COMPRISING GUANIDINO GROUPS AND USE THEREOF FOR COSMETIC FORMULATIONS
SILOXANES CONTENANT DES GROUPES GUANIDINO ET UTILISATION POUR DES FORMULATIONS COSMETIQUES

(30) Priorität: 02.02.2005 DE 102005004704
(43) Veröffentlichungstag der Anmeldung: 17.10.2007
(73) Patentinhaber: Evonik Goldschmidt GmbH, 45127 Essen (DE)
(72) Erfinder: PASCALY, Matthias, 48153 Münster (DE); LEIDREITER, Holger, 45529 Hattingen (DE); FERENZ, Michael, 45359 Essen (DE)
(74) Vertreter: Lang, Arne
(86) Internationale Anmeldenummer: PCT/EP2006/000260
(87) Internationale Veröffentlichungsnummer: WO 2006/081927

(56) Entgegenhaltungen:
- DE-A1- 1 694 382
- US-A1- 2003 235 552
- US-A1- 2004 258 651
- PATENT ABSTRACTS OF JAPAN Bd. 2002, Nr. 10, 10. Oktober 2002 (2002-10-10) & JP 2002 167437 A (LION CORP; LION AKZO KK), 11. Juni 2002 (2002-06-11)

## Beschreibung

Menschliches Haar ist täglich den verschiedensten Einflüssen ausgesetzt. Neben mechanischen Beanspruchungen durch Bürsten, Kämmen, Hochstecken oder Zurückbinden, werden die Haare auch durch Umwelteinflüsse wie z.B. starke UV-Strahlung, Kälte, Wind und Wasser angegriffen. Auch der physiologische Status (z.B. Alter, Gesundheit) der jeweiligen Person beeinflusst die Schädigung der keratinischen Fasern.

Insbesondere aber auch die Behandlung mit chemischen Mitteln verändert Struktur und Oberflächeneigenschaften der Haare. Methoden wie z.B. das Dauerwellen, Bleichen, Färben, Tönen, Glätten usw., aber auch häufiges Waschen mit aggressiven Tensiden tragen dazu bei, dass mehr oder weniger starke Schäden an der Haarstruktur verursacht werden. So wird z.B. bei einer Dauerwelle sowohl der Cortex als auch die Cuticula des Haares angegriffen. Die Disulfid-Brücken des Cystins werden durch den Reduktionsschritt aufgebrochen und im anschließenden Oxidationsschritt zum Teil zu Cysteinsäure oxidiert.

Beim Bleichen wird nicht nur das Melanin zerstört, sondern es werden außerdem ca. 15 bis 25 Gew.-% der Disulfid-Bindungen des Cystins bei einer milden Bleiche oxidiert. Bei einer exzessiven Bleichung können es sogar bis zu 45 Gew.-% sein (K. F. de Polo, A Short Textbook of Cosmetology, 2000, Verlag für chemische Industrie, H. Ziolkowsky GmbH).

So ergeben sich aus den chemischen Behandlungen, dem häufigen Waschen oder der UV-Bestrahlung nachteilige mechanische Eigenschaften für das Haar, hervorgerufen durch Entfernung natürlich ausgeschiedener Haarfette bzw. -feuchthaltemittel (Sebum). Es wird dadurch spröde, trocken, glanzlos, porös und schlecht kämmbar. Außerdem ist gründlich gereinigtes Haar für gewöhnlich sehr schwer zu kämmen, sowohl in nassem als auch in trockenem Zustand, da die einzelnen Haare dazu neigen kraus zu werden und sich zu verknoten. So verliert es erst beim Waschen und anschließend beim Kämmen seine Widerstandsfähigkeit. Dies zeigt sich in einer signifikanten Abnahme der Zug-Dehnungskräfte und der Reißkräfte bei nassem Haar. Außerdem ist es gegenüber einer weiteren Schädigung durch Chemikalien, Tenside und Umwelteinflüsse weniger widerstandsfähig als gesundes Haar.

Für die Reparatur derart geschädigter Haare gibt es spezielle Zubereitungen, wie z.B. Haarspülungen, Haarkuren, Shampoos, Leave-in Konditionierer usw., die jedoch vor allem die Kämmbarkeit, den Griff und den Glanz geschädigter Haare verbessern können. Derartige handelsübliche Haarpflegemittel enthalten hauptsächlich kationische Tenside auf Alkylammonium-Basis, Polymere, Wachse bzw. Öle oder Siliconöle. Die Wirksamkeit dieser Verbindungen lässt sich auf eine elektrostatische Wechselwirkung der kationischen Quat-Gruppen bzw. auf eine Hydrophobisierung der Haar-Oberfläche zurückführen. Eine (bio)chemische Reparatur des Haares wird dadurch jedoch nicht erreicht.

Dem speziellen Problem, dass bei Haaren durch Schädigung die mechanische Widerstandsfähigkeit stark reduziert wird, wird schon seit langem große Aufmerksamkeit geschenkt.

In diesem Zusammenhang ist beispielsweise der Einsatz von Kreatin bekannt. In DE-A-101 14 561 und DE-A-101 19 608 wird die Verwendung von Kreatin-Verbindungen in Mitteln zur Härtung, Stärkung, Restrukturierung oder Erhöhung von Glanz, Volumen oder Kämmbarkeit von keratinischen Fasern, insbesondere menschlichen Haaren beschrieben.

Ein Nachteil bei der Verwendung von Kreatin ist die Tatsache, dass Kreatin nur über die wässrige Phase formuliert werden kann und es sich in wässrigen Lösungen immer in einem Gleichgewicht mit Kreatinin befindet und somit als aktive Substanz für das Haar nicht mehr zur Verfügung steht. Des Weiteren wird es als monomere Verbindung mit geringem Molekulargewicht leicht aus dem zu behandelnden Gewebe bzw. der Oberfläche (Haut oder Haare) wieder ausgewaschen.

Auch die Verwendung kreatinartiger Verbindungen für die Verwendung in Haarbehandlungsmitteln und Haarnachbehandlungsmitteln zur Reparatur und Konditionierung wird in der nicht veröffentlichten Literatur DE-103 27 871.0 und EP-03013799.6 beschrieben. Diese und zahlreiche weitere Schriften (DE-506 282, JP-A-6-263621, JP-11035424, JP-10017442) beschreiben die unterschiedlichsten Methoden zur Herstellung verschiedenster Alkylguanidine. Nur in der JP-2002167437 wird, ausgehend von Amodimethiconen, die Herstellung und Verwendung einfacher linearer siliconhaltiger Guanidine beschrieben. Diese werden zur Haarbehandlung mit Haarkosmetika und für Faserbehandlungsmittel eingesetzt.

Weitere Wirkungen und Verwendungen Guanidinium-haltiger Siliconverbindungen sind in der Literatur bislang nicht bekannt. Auch Kombinationen unterschiedlicher funktioneller Gruppen in Siliconen neben Guanidino-Gruppen sind nicht bekannt. Außerdem ist die Herstellung Guanidinium-haltiger Silicon-Monomerer und deren anschließende Polymerisation unbekannt.

Vor diesem Hintergrund ist es von großer Wichtigkeit weitere neue Substanzen zu identifizieren, die eine ähnliche physiologische Wirkung erzielen oder auch die Wirkung von Kreatin unterstützen und verstärken, eine polymere Struktur besitzen und zusätzlich zu den vorteilhaften Eigenschaften der Polysiloxane die Haare konditionierende, schützende und wiederherstellende (repair) Effekte aufweisen. So können in diesem Rahmen neben Guanidino-Gruppen auch weitere funktionelle Gruppen in das Silicon eingeführt werden.

Es besteht daher also weiterhin ein Bedarf an vielseitig einsetzbaren Wirkstoffen für Haarbehandlungsmittel und Haarnachbehandlungsmittel, die die mechanische Widerstandsfähigkeit des Haares verbessern, das Haar vor weiterer Schädigung der Haarstruktur schützen und die bereits verursachten strukturellen Schädigungen des Haares, hervorgerufen durch Umwelteinflüsse sowie form- und farbgebende Behandlungen, zu minimieren und die auf einfache Art und Weise herstellbar sind und es erlauben, verschiedene funktionelle Gruppen unabhängig voneinander zu kombinieren.

Es ist eine Aufgabe der Erfindung, einen solchen Wirkstoff zur Verfügung zu stellen, der in der Lage ist, sowohl die mechanische Widerstandsfähigkeit von geschädigten Haaren zu verbessern, als auch das Haar vor Schädigungen durch eine chemische Behandlung oder exogenen Faktoren zu schützen. Die Verbindungen sollen also eine kombinierte Wirkung zeigen. Zum einen sollen sie als Konditioniermittel wirken und den Glanz, die Kämmbarkeit, die Weichheit, das Volumen, die Formbarkeit, die Handhabbarkeit und die Entwirrbarkeit der Haare verbessern und gleichzeitig sollen die erfindungsgemäßen Verbindungen die Haare vor Schädigung schützen und widerstandsfähiger machen bzw. diese reparieren. Dabei sollen sich die Eigenschaften der Verbindungen gezielt einstellen lassen durch freie Kombination der Substituenten am Silicon-Backbone.

Ein Gegenstand der Erfindung sind daher Guanidinogruppen enthaltende Polysiloxane der allgemeinen Formel (I) 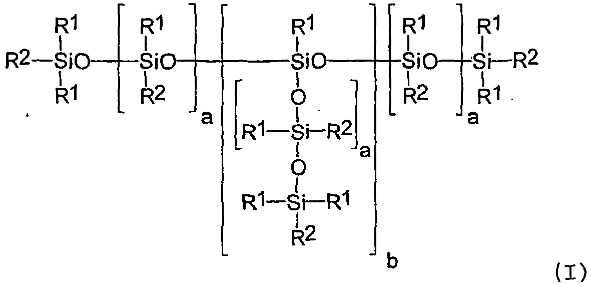 worin die Reste
- R¹: gleich oder verschieden sind und ein gegebenenfalls verzweigter, gegebenenfalls Doppelbindungen enthaltender Kohlenwasserstoffrest mit 1 bis 30 C-Atomen, vorzugsweise 1 bis 4 C-Atomen oder Phenylreste oder -OR¹¹ oder -OH bedeuten,
- R²: zu einem Teil die Bedeutung der Reste R¹ haben können und die übrigen Reste R² unabhängig voneinander Reste der Formeln (Ia, Ib oder Ic)

R² = -M-G (Ia)

R² = -M-Q⁺ A⁻ (Ib)

R² = - (M)ₓ-S (Ic)

sind, mit der Maßgabe, dass im durchschnittlichen Molekül mindestens ein Rest R² ein Rest der Formel -M-G (Ia) ist,
worin
G eine Guanidinogruppe mit der allgemeinen Formel (Ia¹, Ia²) 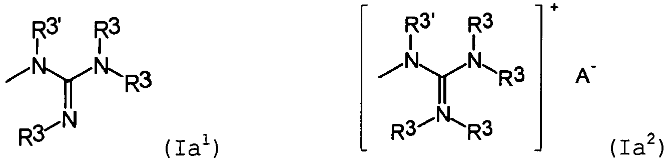 und/oder deren Salze oder Hydrate ist, in denen
R³ a) unabhängig voneinander, Wasserstoff oder ein gegebenenfalls verzweigter, gegebenenfalls Doppelbindungen enthaltender Kohlenwasserstoffrest, oder
R³' R³ sein kann oder eine Alkylengruppe, die mit M über Kohlenstoff- oder Heteroatome verknüpft ist und so einen 5 bis 8-gliedrigen Ring bildet und
M ein zwei- oder mehrwertiger Kohlenwasserstoffrest mit mindestens 4 Kohlenstoffatomen ist, der eine Hydroxylgruppe aufweist und der durch ein oder mehrere Sauerstoffatome oder Stickstoffatome oder quaternäre Ammoniumgruppen oder Ester oder Amidfunktionen unterbrochen ist,
Q⁺ ein Rest der Formel (Id) 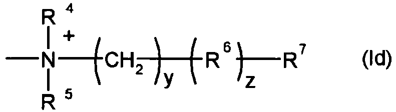 ist,
R⁴, R⁵ Alkylreste mit 1 bis 4 Kohlenstoffatomen sind,
R⁶ 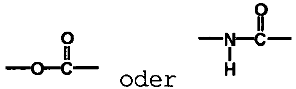 ist,
R⁷ ein einwertiger Kohlenwasserstoffrest mit 1 bis 22 Kohlenstoffatomen sein kann,
y 0 bis 6
z 0 oder 1,
A⁻ ein anorganisches oder organisches Anion, das von einer üblichen physiologisch verträglichen Säure HA herrührt,
S H, ein Polyalkylenoxid-Polyether der allgemeinen Formel CₘH₂ₘO(C₂H₄O)ₙ(C₃H₆O)ₒR⁸
worin
m 1 bis 6, insbesondere 3, 6,
n,o unabhängig voneinander 0 bis 100, insbesondere 0 bis 20 und der Polyether ein Molekulargewicht zwischen 100 und 6.000 g/mol aufweist und
R⁸ H oder ein gegebenenfalls verzweigter, gegebenenfalls Doppelbindungen enthaltender aromatischer oder alicyclischer Kohlenwasserstoffrest mit 2 bis 30 C-Atomen, vorzugsweise 4 bis 22 C-Atomen, oder eine UV-absorbierende Gruppe, insbesondere Zimtsäure oder Methoxyzimtsäure,
x 0 oder 1 ist und
- a: unabhängig voneinander einen Wert von 8 bis 1.000 vorzugsweise 20 bis 200 und
- b: einen Wert von 0 bis 10 hat.

Bevorzugte Beispiele für A⁻, ein anorganisches oder organisches Anion, stammen von einer üblichen physiologisch verträglichen Säure HA wie Ameisensäure, Essigsäure, Propionsäure, Heptansäure, Caprylsäure, Nonansäure, Caprinsäure, Undecansäure, Laurinsäure, Myristinsäure, Palmitinsäure, Stearinsäure, Arachinsäure, Behensäure, Cyclopentancarbonsäure, Cyclohexancarbonsäure, Acrylsäure, Methacrylsäure, Vinylessigsäure, Crotonsäure, 2-/3-/4-Pentensäure, 2-/3-/4-/5-Hexensäure, Lauroleinsäure, Myristoleinsäure, Palmitoleinsäure, Ölsäure, Gadoleinsäure, Sorbinsäure, Linolsäure, Linolensäure, Pivalinsäure, Ethoxyessigsäure, Phenylessigsäure, Milchsäure, 2-Ethylhexansäure, Oxalsäure, Glycolsäure, Äpfelsäure, Malonsäure, Bernsteinsäure, Weinsäure, Glutarsäure, Zitronensäure, Adipinsäure, Pimelinsäure, Korksäure, Azelainsäure, Sebacinsäure, Benzoesäure, o-/m-/p-Tolylsäure, Phenylessigsäure, Salicylsäure, 3-/4-Hydroxybenzoesäure, Phthalsäuren oder deren ganz oder teilweise hydrierten Derivate wie Hexahydro- oder Tetrahydrophthalsäure, Kohlensäure, Phosphorsäure, Salzsäure, Schwefelsäure und deren Gemische, insbesondere Milchsäure, Weinsäure, Essigsäure und Salzsäure. Dabei ist es im Sinne der vorliegenden Erfindung auch möglich, sowohl geeignete Silicon-Guanidin-Derivate untereinander in Mischungen zu verwenden als auch Mischsalze.

Desweiteren sind Gegenstand dieser Erfindung Silicon-Guanidin-Derivate die erhalten werden durch die Umsetzung von Guanidino-gruppen-haltigen Silanen der allgemeinen Formel II mit einem oder mehreren verschiedenen Silanen der allgemeinen Formel III und einem oder mehreren verschiedenen OH- , oder OR¹¹-funktionellen Siloxanen der allgemeinen Formel IV, gegebenenfalls in Gegenwart eines die Kondesation fördernden Katalysators und/oder Wasser und Emulgatoren 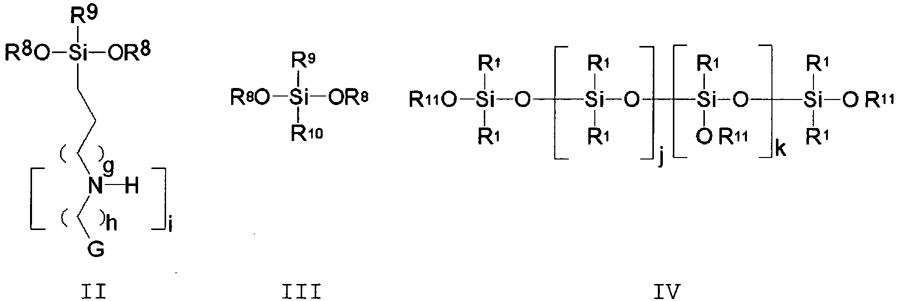 worin
- R¹: gleich oder verschieden sind und ein gegebenenfalls verzweigter, gegebenenfalls Doppelbindungen enthaltender Kohlenwasserstoffrest mit 1 bis 30 C-Atomen oder Phenylreste oder -OR¹¹ oder -OH bedeuten,
- R⁸: gleich oder verschieden sind und Kohlenwasserstoffreste mit 1 bis 30 C-Atomen, vorzugsweise Kohlenwasserstoffreste mit 1 bis 2 C-Atomen oder Phenylreste darstellen,
- R⁹: gleich oder verschieden sind und Kohlenwasserstoffreste mit 1 bis 30 C-Atomen, vorzugsweise Kohlenwasserstoffreste mit 1 bis 2 C-Atomen oder Phenylreste, oder OR⁸-Reste darstellen,
- g: 0 bis 10, vorzugsweise 1 bis 3,
- h: 1 bis 11, vorzugsweise 1 bis 4,
- i: 0 bis 3, vorzugsweise < 2
- R¹⁰: aliphatische oder aromatische Kohlenwasserstoffreste mit 1 bis 30 C-Atomen sind, die gegebenenfalls verzweigt sind und eine oder mehrere Amin- oder OH- oder SH-Gruppen tragen und gegebenenfalls durch Amin- oder Etherfunktionen unterbrochen oder OR⁸ oder Polyether sind.
- R¹¹: aliphatische oder aromatische Kohlenwasserstoffreste mit 1 bis 30, vorzugsweise 1 bis 4 C-Atomen sind, die gegebenenfalls verzweigt oder H sind.
- k: eine ganze Zahl zwischen 0 und 10,
- j: eine ganze Zahl zwischen 8 und 1.000 ist.

Ein weiterer Gegenstand der Erfindung ist ein Verfahren zur Herstellung dieser Verbindungen, welches dadurch gekennzeichnet ist, dass ein mindestens eine Epoxy-Gruppe enthaltendes Siloxan bei erhöhter Temperatur mit Guanidin-Verbindungen der allgemeinen Formel

Z-G,

worin
- G: die oben angegebene Bedeutung hat und
- Z: ein mindestens 2 Kohlenstoffatome, vorzugsweise 3 bis 6, enthaltender Kohlenwasserstoffrest, gegebenenfalls Heteroatome enthaltend, insbesondere N, der mit R³' über ein Kohlenstoff- oder Heteroatom verbunden sein kann und so einen 5 bis 8-gliedrigen Ring bildet, ist,
und/oder deren Salze nach an sich bekannten Verfahren umgesetzt werden.

Ein weiterer Gegenstand der Erfindung ist ein Verfahren zur Herstellung dieser Verbindungen, welches dadurch gekennzeichnet ist, dass Guanidinogruppen-haltige Silane der allgemeinen Formel II mit einem oder mehreren verschiedenen Silanen der allgemeinen Formel III und einem oder mehreren verschiedenen OH- oder OR¹¹-funktionellen Siloxanen der allgemeinen Formel IV, in Gegenwart eines die Kondesation fördernden Katalysators gemischt und erhitzt werden, gegebenenfalls in Gegenwart von Wasser und einen oder mehreren Emulgatoren.

Es ist dem Fachmann geläufig, dass die Verbindungen in Form eines Gemisches mit einer im Wesentlichen durch statistische Gesetze geregelten Verteilung vorliegen. Die Werte für die Indices a und b stellen deshalb Mittelwerte dar.

Ein weiterer Gegenstand der Erfindung ist die Verwendung der erfindungsgemäßen Verbindungen als Konditioniermittel zur Herstellung von kosmetischen Formulierungen zur Haarbehandlung und Haarnachbehandlung, die gleichzeitig eine Verbesserung der Haarstruktur bewirken.

Die erfindungsgemäß verwendeten oder mitverwendeten Silicon-guanidine besitzen sowohl eine gute Stabilität als auch eine gute Formulierbarkeit, rufen bereits in geringen Einsatzkonzentrationen eine deutliche Wirkung hervor und sind nicht toxisch, werden sehr gut vom Haar und der Kopfhaut toleriert, weisen eine hohe Verträglichkeit mit anderen Inhaltsstoffen auf und lassen sich problemlos in Haarbehandlungsmittel einarbeiten. Zusätzlich können sie noch eine leicht antimikrobielle Wirkung aufweisen.

Die erfindungsgemäßen Verbindungen kombinieren also verschiedene funktionelle Gruppen am Silicone-Backbone. Gerade durch diese Kombinationen ergibt sich für das Haar neben dem konditionierenden Effekt auch eine Verbesserung der Faserfestigkeit (Dehnungsfestigkeit und Elastizitätsmodul werden erhöht). Diese Wirkung wird allgemein auch als Reparatur-Effekt bezeichnet. Durch die hohe Affinität der Guanidingruppe zum Keratin des Haares wird eine nachhaltige Wirksamkeit beobachtet. Die Eigenschaften der Verbindungen lassen sich nach einem Baukasten-Prinzip unabhängig voneinander gezielt einstellen und aufeinander abstimmen.

In Pflegeformulierungen für poröse Oberflächen wie Leder oder Möbel erhält man mit Siliconguanidinen eine sehr gute Glanzsteigerung auf den behandelten Oberflächen. Durch ihre Substantivität geben sie einen lang anhaltenden Schutzfilm, der die Wiederanschmutzung durch seinen antistatischen Effekt verhindert. Durch ihre hydrophoben Eigenschaften verhindern sie zudem ein schnelles Durchnässen poröser Materialien. Solche Pflegeemulsionen enthalten üblicherweise 1 bis 3 % Emulgatoren, 3 bis 5 % Wachse, 2 bis 5 % Öle und 0,5 bis 1 % Dickungsmittel in wässriger Lösung. Hierzu werden 0 bis 5 % und besonders 1 bis 3 % der erfindungsgemäßen Siliconguanidinverbindung eingesetzt um die oben genannten Eigenschaften deutlich zu erhöhen.

In Pflegeformulierungen für glatte Oberflächen wie metallische oder lackierte Oberflächen erhält man mit Siliconguanidinen eine sehr gute Glanzsteigerung auf den behandelten Oberflächen ohne eine zu starke Fettigkeit der Oberflächen zu erzielen. Durch ihre Substantivität geben sie einen lang anhaltenden Schutzfilm, der die Wiederanschmutzung durch seinen antistatischen Effekt verhindert. Die Verwendung des Siliconguanidins reduziert die üblichen Pflegestoffe und somit werden auch die mit Fingerprint bezeichneten Abdrücke reduziert, die bei Verwendung von Pflegestoffen wie Ölen oder Wachsen auftreten. Pflegeemulsionen für glatte Oberflächen enthalten üblicherweise 0 bis 10 % Emulgatoren, 1 bis 8 % Wachse, 2 bis 8 % Öle, zumeist Siliconöle und 0,5 bis 1 % Dickungsmittel in wässriger Lösung. Hierzu werden 0 bis 5 % und besonders 1 bis 3 % der erfindungsgemäßen Siliconguanidinverbindung eingesetzt um die oben genannten Eigenschaften deutlich zu erhöhen.

In Trocknungshilfen für Autowaschstraßen werden die erfindungsgemäßen Siliconguanidinverbindungen den hier üblichen Mikroemulsionen zugesetzt, um auch hier durch ihre Substantivität einen lang anhaltenden Schutzfilm zu erzeugen. Dieser verhindert die Wiederanschmutzung durch seinen antistatischen Effekt und erzeugt somit einen langanhaltenden Glanz. Übliche Trocknungshilfen enthalten 0,1 bis 5 % der erfindungsgemäßen Siliconguanidinverbindung um diesen Effekt zu erzeugen. Die beschriebene Trocknungshilfe ist eine kationische Mikroemulsion aufgebaut aus einer oder mehrerer quaternärer Verbindungen, Einsatzmenge in der Regel 5 bis 25 % und einem oder mehreren hydrophoben Ölen, Einsatzmenge in der Regel 3 bis 30 %, die gegebenenfalls mit Co-emulgatoren, Einsatzmenge in der Regel 0 bis 5 %, stabilisiert werden.

Bevorzugte Beispiele für Guanidinium-Gruppen enthaltende Polysiloxane, sind Verbindungen der allgemeinen Formel (I)

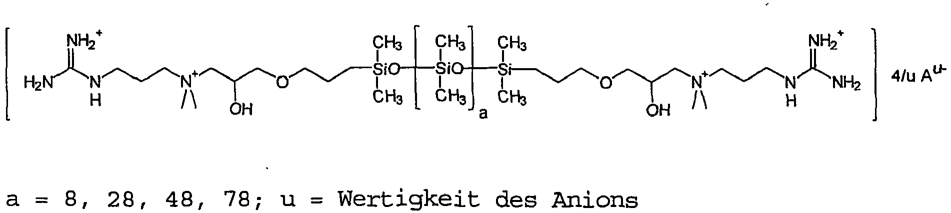

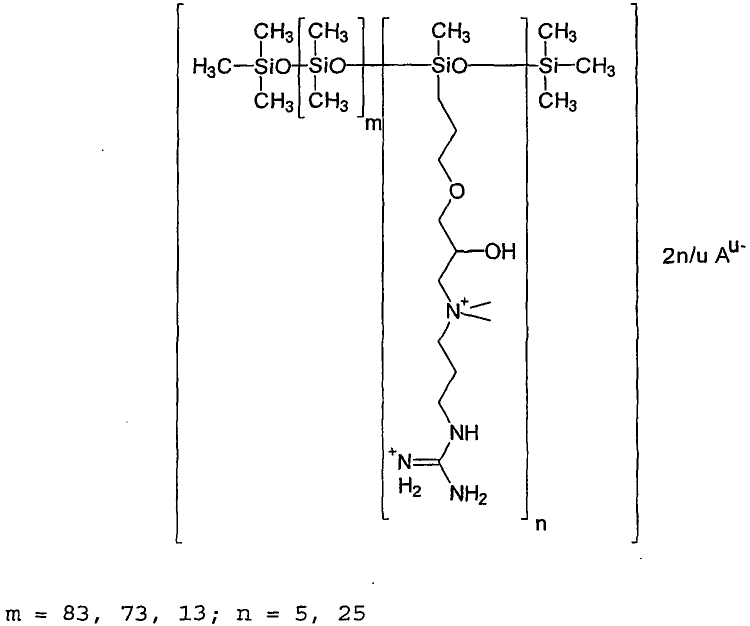

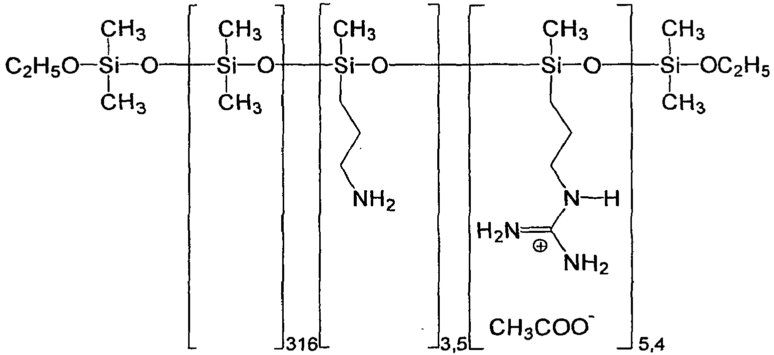

Verfahren zur Herstellung der erfindungsgemäßen Verbindungen und die Eigenschaften dieser Verbindungen sind in den folgenden Beispielen näher beschrieben:

Die Synthese einer Vorstufe (1-(3-N,N-Aminopropyl-)Guanidinium-Acetat) zur Herstellung quaternärer Silicon-Guanidine erfolgte nach einer Vorschrift beschrieben in der DE-506 282 bzw. DE-103 27 871.0 und EP-03013799.6.

### Beispiel 1:

Herstellung eines erfindungsgemäßen Guanidino-Polysiloxans der allgemeinen Formel:

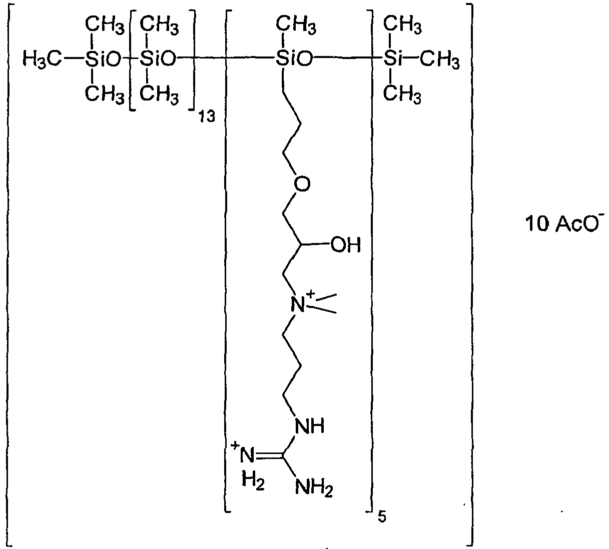

In einem 250 ml-Vierhalskolben, versehen mit Rührer, Tropftrichter, Thermometer und Rückflusskühler, werden 10 g (0,1 mol) einer Guanidinium-Gruppe (vorliegend als Acetat) enthaltenden tertiären Amins der Formel 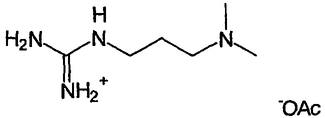 zusammen mit 3 g (0,103 mol) Essigsäure und 40 ml Isopropanol vorgelegt. Nach ca. 1 Stunde werden 19,5 g (0,1 mol Epoxy) eines Epoxysiloxans (diese sind hergestellt nach bekanntem Verfahren durch Hydrosilylierung mit Allylglycidylether) der Formel 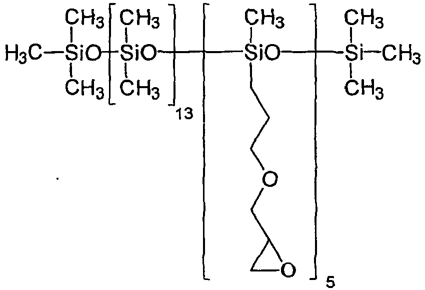 zugetropft, auf Rückflusstemperatur erwärmt und 6 Stunden gerührt. Anschließend wird bei 80 °C im Vakuum destilliert. Es wird ein hochviskoses, gelbes Produkt erhalten.

### Beispiel 2:

Herstellung eines erfindungsgemäßen Guanidino-Polysiloxans der allgemeinen Formel:

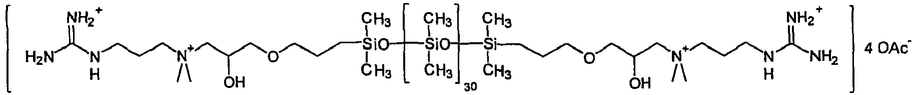

In einem 250 ml-Vierhalskolben, versehen mit Rührer, Tropftrichter, Thermometer und Rückflusskühler, werden 20,4 g (0,1 mol) des bereits in Beispiel 1 erwähnten tertiären Amins zusammen mit 6,2 g (0,1 mol) Essigsäure in 40 ml Isopropanol vorgelegt. Nach 1 Stunde unter Rückflußtemperatur werden 113 g (0,1 mol Epoxy) eines Epoxysiloxans der allgemeinen Formel 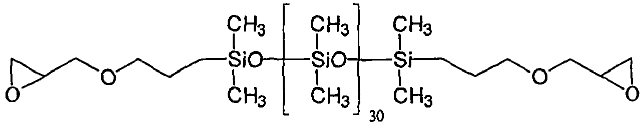 zugetropft, auf Rückflusstemperatur erwärmt und 6 Stunden gerührt. Anschließend wird bei 100 °C im Vakuum destilliert. Es wird ein wachsartiges, gelbes Produkt erhalten.

### Beispiel 3:

Herstellung eines erfindungsgemäßen Guanidino-Polysiloxans der allgemeinen Formel:

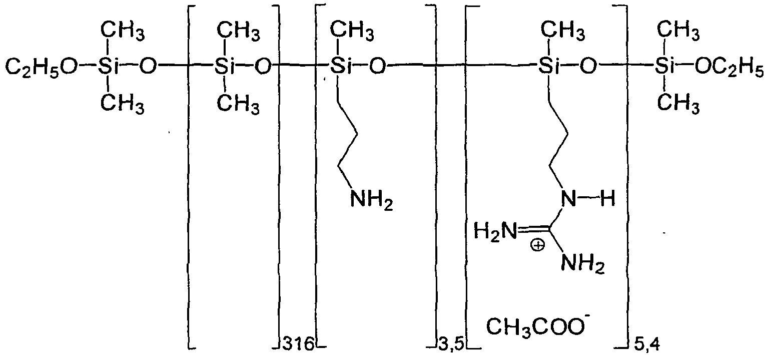

In einem 250 ml-Kolben werden 70 g Wasser, 40 g Fettalkoholethoxylat, 10 g 50 %ige wässrige Natronlauge, 10 g eines Guanidinogruppen-haltigen Silans MeSi (OEt)₂- (CH₂)₃-NHC (NH₂) =NH₂⁺ CH₃COO⁻, 10 g eines aminofunktionellen Siloxans MeSi (OEt) ₂- (CH₂) -NH₂ und 240 g eines Silanols der Formel HO-SiMe₂O-(SiMe₂O)-SiMe₂OH miteinander verrührt. Man heizt auf 70 °C und lässt 4 Stunden reagieren. Anschließend wird mit 130 ml Wasser verdünnt und mit Essigsäure neutralisiert. Man erhält eine wässrige Emulsion des oben angegebenen Guanidinogruppen-haltigen Siloxans.

### Anwendungstechnischer Vergleich:

Für den anwendungstechnischen Vergleich wurden folgende erfindungsgemäße Guanidino-Polysiloxane (vorliegend mit Acetat als Gegenion) eingesetzt:

### Verbindungen 1 und 2 gemäß Strukturformel:

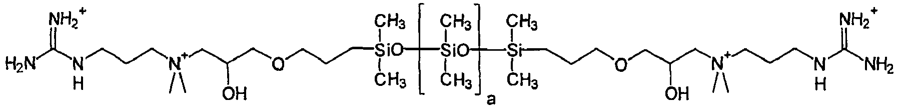

wobei Verbindung 1 mit a = 8 und
Verbindung 2 mit a = 28 ist.

### Verbindungen 3 und 4 gemäß Strukturformel:

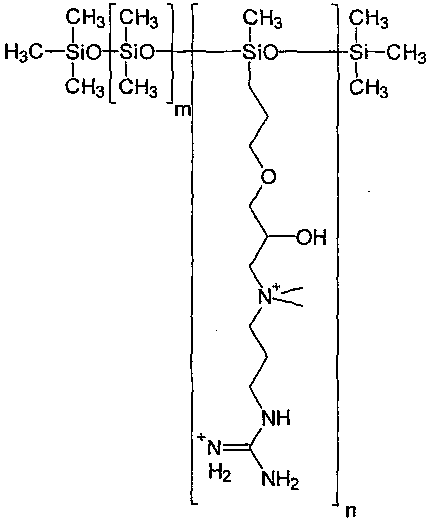

wobei Verbindung 3 mit m = 13 und n = 5 und
Verbindung 4 mit m = 83 und n = 5 ist.

Herstellung und Überprüfung von Haarbehandlungsmitteln unter Verwendung der erfindungsgemäßen Verbindungen 1, 2 und 3:

Für die anwendungstechnische Beurteilung werden Haartressen, die für sensorische Tests verwendet werden, durch eine Dauerwellbehandlung und eine Bleichbehandlung standardisiert vorgeschädigt. Dazu werden friseurübliche Produkte verwendet.

### Materialien:

- Dauerwell-Flüssigkeit (z.B. "ondi", Wella)
- Fixierung (z.B. "neutrafix", Wella)
- Blondierpulver (z.B. "blondor special", Wella)
- H₂O₂ (e.g. "Welloxyd 9 %", Wella)
- Shampoo ohne Pflegeanteil (z.B. Natriumlaurylethersulfat (12 % WAS), NaCl verdickt)
- Bechergläser
- Haar-Färbe-Pinsel

Die Behandlung wird in der folgenden Reihenfolge durchgeführt:
1. Dauerwell-Behandlung:
   Die Haartressen werden mit der Dauerwellflüssigkeit angefeuchtet (Gewichtsverhältnis Haar : Flüssigkeit = 1 : 2). Nach einer Einwirkzeit von 15 min bei Raumtemperatur in einem abgedeckten Becherglas wird die Dauerwellflüssigkeit 2 min sorgfältig ausgespült. Anschließend werden die Haarsträhnchen mit einem Handtuch sanft ausgedrückt.
   Die Fixierung (Verhältnis Haar : Flüssigkeit = 1 : 2) hat eine Einwirkzeit von 10 min bei Raumtemperatur. Anschließend wird die Fixierung sorgfältig 2 min ausgespült.
   Die Haare werden anschließend über Nacht bei Raumtemperatur getrocknet.
2. Bleich-Behandlung:
   Das Blondierpulver und das H₂O₂ werden zu einer Paste (Gewichtsverhältnis Pulver: H₂O₂ = 2 : 3) verarbeitet. Dann wird die Paste sofort auf die dauergewellten Haare mit einem Pinsel aufgetragen. Die Einwirkzeit beträgt 30 min bei Zimmertemperatur. Anschließend wird die Blondierpaste unter fließendem Wasser 2 min ausgespült.
   Dann wird das Haar mit einem Shampoo ohne Conditioner 1 min gewaschen (Shampoomenge: 0,5 ml/Hairtress) und dann für 1 min ausgespült.
   Bevor die vorgeschädigten Haarsträhnchen für Sensorik-Tests verwendet werden, werden sie über Nacht bei Raumtemperatur getrocknet.

### Testrezeptur:

Die konditionierenden Produkte werden in einer einfachen Haarspülung mit folgendem Aufbau

| Produkt | Gewichtsanteile |
|---|---|
| TEGINACID^{®}C Ceteareth-25 | 0,5 % |
| TEGO^{®}Alkanol 16 Cetylalkohol | 2,0 % |
| Konditioniermittel | 2,0 % |
| Wasser | ad. 100 % |
| Zitronensäure | ad. pH 4,0 ± 0,3 |

getestet.

Als "Konditioniermittel" sind die erfindungsgemäßen Verbindungsbeispiele oder Vergleichsprodukte bezeichnet. Standardisierte Behandlung von vorgeschädigten Haarsträhnen mit konditionierenden Proben:

Die, wie oben beschrieben, vorgeschädigten Haarsträhnchen werden wie folgt mit der oben beschriebenen konditionierenden Spülung behandelt:

Die Haarsträhnen werden unter fließendem, warmem Wasser benetzt. Das überschüssige Wasser wird leicht von Hand ausgedrückt, dann wird die Spülung aufgebracht und sanft im Haar eingearbeitet (1 ml/Haarsträhne (2 g)). Nach einer Verweilzeit von 1 min wird das Haar für 1 min gespült.

Vor der sensorischen Beurteilung wird das Haar an der Luft bei 50 % Luftfeuchtigkeit und 25 °C für mindestens 12 h getrocknet.

### Beurteilungskriterien:

Die sensorischen Bewertungen erfolgen nach Noten, die auf einer Skala von 1 bis 5 vergeben werden, wobei 1 die schlechteste und 5 die beste Bewertung ist.

### Nasskämmbarkeit:

| Bewertung | Zahnung des Kamms | Ergebnis |
|---|---|---|
| 5 | grob | keine Knoten, das Haar ist leicht entwirrbar |
| | fein | sehr leichtes Durchkämmen, kein Widerstand spürbar |
| 4 | grob | einzelne Knoten, das Haar ist leicht entwirrbar |
| | fein | leichtes Durchkämmen, geringer Widerstand spürbar |
| 3 | grob | wenige Knoten, geringer Widerstand |
| | fein | etwas Widerstand spürbar, der nach mehrmaligem Kämmen nachlässt |
| 2 | grob | einige Knoten, merklicher Widerstand |
| | fein | merklicher Widerstand, der nach mehrmaligem Kämmen nicht nachlässt. |
| 1 | grob | viele Knoten, starker Widerstand |
| | fein | sehr starker Widerstand, manchmal ist das Haar nicht durchkämmbar |

### Nassgriff:

| Bewertung | Ergebnis |
|---|---|
| 5 | sehr glatt, weich aber trotzdem schön fest, griffig, nicht schmierig/klebrig (keine Rückstände fühlbar) |
| 4 | glatt und weich und/oder nur geringe Rückstände spürbar |
| 3 | glatt, etwas hart und/oder etwas Rückstände spürbar |
| 2 | hart und/oder merkliche schmierige, wachsige Rückstände |
| 1 | sehr hart, rauh, stumpf und/oder extrem schmierig, klebrig (deutlich spürbare schmierige, wachsige Rückstände spürbar) |

### Trockenkämmbarkeit:

| Bewertung | Zahnung des Kamms | Ergebnis |
|---|---|---|
| 5 | grob | keine Knoten, das Haar ist leicht entwirrbar |
| | fein | sehr leichtes Durchkämmen, kein Widerstand spürbar, das Haar lädt sich nicht auf |
| 4 | grob | einzelne Knoten, das Haar ist leicht entwirrbar |
| | fein | leichtes Durchkämmen, geringer Widerstand spürbar, Haar lädt sich minimal auf |
| 3 | grob | wenige Knoten, geringer Widerstand |
| | fein | etwas Widerstand spürbar, der nach mehrmaligem Kämmen nachlässt, Haar lädt sich wenig auf |
| 2 | grob | einige Knoten, merklicher Widerstand |
| | fein | merklicher Widerstand, der nach mehrmaligem Kämmen nicht nachlässt, Haar lädt sich auf |
| 1 | grob | viele Knoten, starker Widerstand |
| | fein | sehr starker Widerstand, manchmal ist das Haar nicht durchkämmbar, Haar lädt sich deutlich auf |

### Trockengriff:

| Bewertung | Ergebnis |
|---|---|
| 5 | sehr glatt, weich aber trotzdem fest, voll, griffig |
| 4 | glatt und weich |
| 3 | glatt, leicht hart und/oder leicht stumpf (Rückstände) |
| 2 | hart, etwas stumpf |
| 1 | rauh, hart, trocken, stumpf (Rückstände) |

### Trocken-Aussehen:

| Bewertung | Ergebnis |
|---|---|
| 5 | extrem glänzend |
| 4 | glänzend |
| 3 | etwas glänzend |
| 2 | wenig glänzend, leicht stumpf |
| 1 | stumpf, kein Glanz |

In der folgenden Tabelle 1 werden die Ergebnisse der sensorischen Beurteilung der wie oben beschrieben durchgeführten Behandlung der Haarstränchen mit erfindungsgemäßen Substanzen bzw. Placebo verglichen.

**Tabelle 1:**

| Formulierung "einfache Haarspülung" mit | Naßkämmbarkeit | Naßgriff | Trockenkämmbarkeit | Trockengriff | Glanz |
|---|---|---|---|---|---|
| Erfindungsgemäße Verbindung 1 | 4,5 | 4,25 | 3,75 | 4,0 | 3,75 |
| Erfindungsgemäße Verbindung 2 | 5,0 | 4,75 | 3,5 | 4,25 | 4,25 |
| Erfindungsgemäße Verbindung 3 | 4,5 | 4,25 | 3,5 | 4,25 | 4,0 |
| Erfindungsgemäße Verbindung 4 | 4,0 | 4,5 | 4,0 | 4,75 | 4,5 |
| Vergleichsverbindung Cetrimonium Chloride | 4,75 | 4,5 | 4,25 | 3,75 | 3,0 |
| Kontrolle ohne Konditioniermittel | 1,5 | 1,5 | 2,25 | 2,75 | 3,5 |

Es zeigt sich, dass die erfindungsgemäßen Verbindungsbeispiele in der sensorischen Beurteilung sehr gute kosmetische Bewertungen erhalten.

## Patentansprüche

1. Guanidinogruppen enthaltende Polysiloxane der allgemeinen Formel (I) 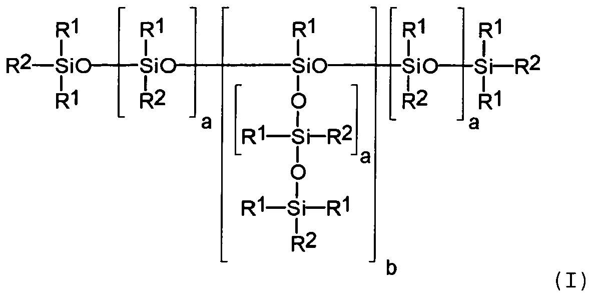 worin die Reste
R¹ gleich oder verschieden sind und ein gegebenenfalls verzweigter, gegebenenfalls Doppelbindungen enthaltender Kohlenwasserstoffrest mit 1 bis 30 C-Atomen oder Phenylreste oder -OR¹¹ oder -OH bedeuten,
R² zu einem Teil die Bedeutung der Reste R¹ haben können und die übrigen Reste R² unabhängig voneinander Reste der Formeln (Ia, Ib oder Ic)
R² = -M-G (Ia)
R² = -M-Q⁺ A⁻ (Ib)
R² = - (M)ₓ-S (Ic)
sind, mit der Maßgabe, dass im durchschnittlichen Molekül mindestens ein Rest R² ein Rest der Formel -M-G (Ia) ist, worin
G eine Guanidinogruppe mit der allgemeinen Formel (Ia¹, Ia²) 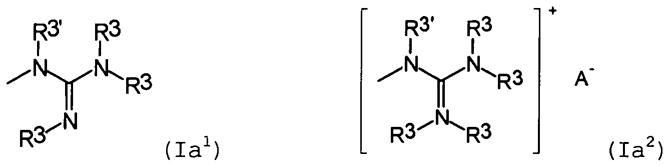
und/oder deren Salze oder Hydrate ist, in denen
R³ a) unabhängig voneinander, Wasserstoff oder ein gegebenenfalls verzweigter, gegebenenfalls Doppelbindungen enthaltender Kohlenwasserstoffrest,
R³' R³ sein kann oder eine Alkylengruppe, die mit M über Kohlenstoff- oder Heteroatome verknüpft ist und so einen 5 bis 8-gliedrigen Ring bildet und
M ein zwei- oder mehrwertiger Kohlenwasserstoffrest mit mindestens 4 Kohlenstoffatomen ist, der eine Hydroxylgruppe aufweist und der durch ein oder mehrere Sauerstoffatome oder Stickstoffatome oder quaternäre Ammoniumgruppen oder Ester- oder Amidfunktionen unterbrochen ist,
Q⁺ ein Rest der Formel (Id) 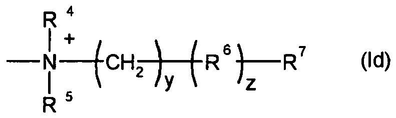 ist,
R⁴, R⁵ Alkylreste mit 1 bis 4 Kohlenstoffatomen sind,
R⁶ 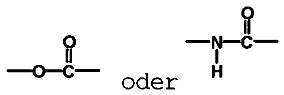 ist,
R⁷ ein einwertiger Kohlenwasserstoffrest mit 1 bis 22 Kohlenstoffatomen sein kann,
y 0 bis 6
z 0 oder 1,
A⁻ ein anorganisches oder organisches Anion, das von einer üblichen physiologisch verträglichen Säure HA herrührt,
S H, ein Polyalkylenoxid-Polyether der allgemeinen Formel CₘH₂ₘO (C₂H₄O)ₙ (C₃H₆O)ₒR⁸ worin
m 1 bis 6, insbesondere 3, 6,
n,o unabhängig voneinander 0 bis 100, insbesondere 0 bis 20 und der Polyether ein Molekulargewicht zwischen 100 und 6.000 g/mol aufweist und
R⁸ H oder ein gegebenenfalls verzweigter, gegebenenfalls Doppelbindungen enthaltender aromatischer oder alicyclischer Kohlenwasserstoffrest mit 2 bis 30 C-Atomen, vorzugsweise 4 bis 22 C-Atomen, oder eine UV-absorbierende Gruppe, insbesondere Zimtsäure oder Methoxyzimtsäure,
x 0 oder 1 ist und
a unabhängig voneinander einen Wert von 8 bis 1.000 vorzugsweise 20 bis 200 und
b einen Wert von 0 bis 10 hat.

2. Verbindungen gemäß Anspruch 1 **dadurch gekennzeichnet, dass** mindestens einer der Reste R² eine Guanidino-Gruppe trägt und R¹ ein Methyl-, Ethyl-, n-Propyl-, iso-Propyl-, n-Butyl-, iso-Butyl-, oder Phenylrest sein kann.

3. Verbindungen gemäß Anspruch 1 und 2 **dadurch gekennzeichnet, dass** M gleich oder verschieden einer der Reste ist, ausgesucht aus der Gruppe

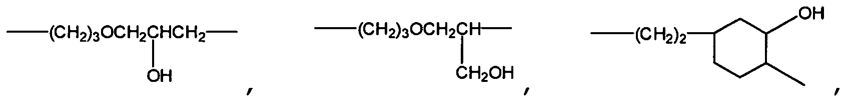

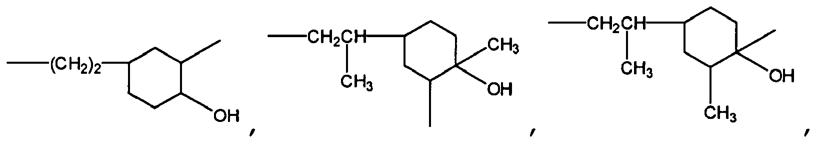

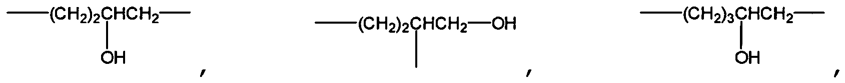

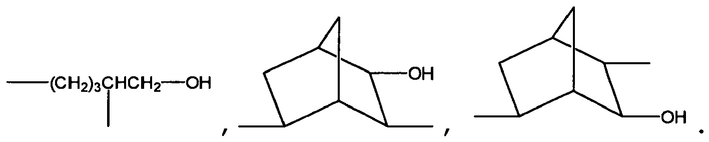

4. Verbindungen gemäß Anspruch 1 **dadurch gekennzeichnet, dass** A⁻, ein anorganisches oder organisches Anion, stammend von einer üblichen physiologisch verträglichen Säure HA wie Kohlensäure, Phosphorsäure, Salzsäure, Schwefelsäure sowie Ameisensäure, Essigsäure, Propionsäure, Heptansäure, Caprylsäure, Nonansäure, Caprinsäure, Undecansäure, Laurinsäure, Myristinsäure, Palmitinsäure, Stearinsäure, Arachinsäure, Behensäure, Cyclopentancarbonsäure, Cyclohexancarbonsäure, Acrylsäure, Methacrylsäure, Vinylessigsäure, Crotonsäure, 2-/3-/4-Pentensäure, 2-/3-/4-/5-Hexensäure, Lauroleinsäure, Myristoleinsäure, Palmitoleinsäure, Ölsäure, Gadoleinsäure, Sorbinsäure, Linolsäure, Linolensäure, Pivalinsäure, Ethoxyessigsäure, Phenylessigsäure, Milchsäure, 2-Ethylhexansäure, Oxalsäure, Glycolsäure, Äpfelsäure, Malonsäure, Bernsteinsäure, Weinsäure, Glutarsäure, Zitronensäure, Adipinsäure, Pimelinsäure, Korksäure, Azelainsäure, Sebacinsäure, Benzoesäure, o-/m-/p-Tolylsäure, Phenylessigsäure, Salicylsäure, 3-/4-Hydroxybenzoesäure, p-Toluolsulfonsäure, Benzoesäure, Salicylsäure, Zimtsäure, 4-Methoxyzimtsäure, 4-Aminobenzoesäure, 4-Bis(hydroxypropyl)aminobenzoesäure, 4-Bis-(polyethoxy)aminobenzoesäure, 4-Dimethylaminobenzoesäure, 3-Imidazol-4-yl-acrylsäure, 2-Phenylbenzimidazol-5-sulfonsäure, 3,3'-(1,4-Phenylendimethin)-bis(7,7-dimethyl-2-oxo-bicyclo[2.2.1]heptan-1-methansulfonsäure), 2-Hydroxy-4-methoxybenzophenon-5-sulfonsäure und 3-(4'-Sulfo)benzyliden-bornan-2-on Phthalsäuren oder deren ganz oder teilweise hydrierten Derivate wie Hexahydro- oder Tetrahydrophthalsäure und deren Gemische, insbesondere Milchsäure, Weinsäure, Essigsäure und Salzsäure.

5. Verbindungen gemäß den Ansprüchen 1 bis 4, **dadurch gekennzeichnet, dass** Q gleich oder verschieden ein kationischer Rest ist, ausgewählt aus der Gruppe Capryldimethylamin, Lauryldimethylamin, Cocodimethylamin, Myristyldimethylamin, Cetyldimethylamin, Stearyldimethylamin, Behenyldimethylamin, Oleyldimethylamin, Capryloylamidopropyldimethylamin, Lauramidopropyldimethylamin, Cocamidopropyldimethylamim, Myristamidopropyldimethylamin, Palmitamidopropyldimethylamin, Stearamidopropyldimethylamin, Behenamidopropyldimethylamin, Oleamidopropyldimethylamin, Undecylenamidopropyldimethylamin, Ricinoleamidopropyldimethylamin sowie Guanidinopropyldimethylamin.

6. Verfahren zur Herstellung der Verbindungen gemäß Anspruch 1 bis 5, **dadurch gekennzeichnet, dass** ein mindestens eine Epoxy-Gruppe enthaltendes Siloxan bei erhöhter Temperatur mit Guanidin-Verbindungen der allgemeinen Formel
Z-G,
worin
G die oben angegebene Bedeutung hat und
Z ein mindestens 2 Kohlenstoffatome, vorzugsweise 3 bis 6, enthaltender Kohlenwasserstoffrest, gegebenenfalls Heteroatome enthaltend, insbesondere N, der mit R³' über ein Kohlenstoff- oder Heteroatom verbunden sein kann und so einen 5 bis 8-gliedrigen Ring bildet, ist,
und/oder deren Salze nach an sich bekannten Verfahren umgesetzt werden.

7. Guanidinogruppen enthaltende Polysiloxane, die erhalten werden durch die Umsetzung von Guanidinogruppen-haltigen Silanen der allgemeinen Formel II mit einem oder mehreren verschiedenen Silanen der allgemeinen Formel III und einem oder mehreren verschiedenen OH- oder OR¹¹-funktionellen Siloxanen der allgemeinen Formel IV, gegebenenfalls in Gegenwart eines die Kondesation fördernden Katalysators und/oder Wasser und Emulgatoren 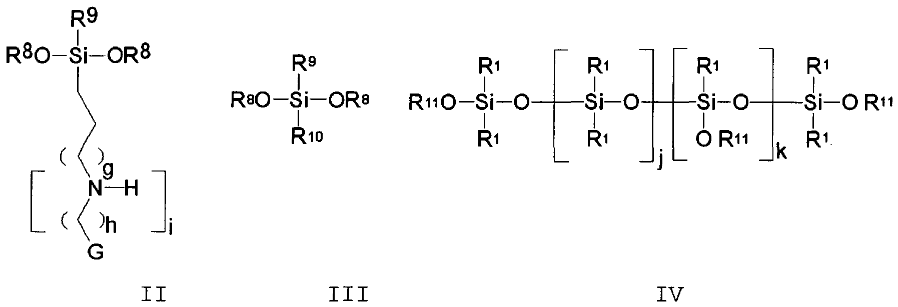 worin
R¹ gleich oder verschieden sind und ein gegebenenfalls verzweigter, gegebenenfalls Doppelbindungen enthaltender Kohlenwasserstoffrest mit 1 bis 30 C-Atomen oder Phenylreste oder -OR¹¹ oder -OH bedeuten,
R⁸ gleich oder verschieden sind und Kohlenwasserstoffreste mit 1 bis 30 C-Atomen, vorzugsweise Kohlenwasserstoffreste mit 1 bis 2 C-Atomen oder Phenylreste darstellen,
R⁹ gleich oder verschieden sind und Kohlenwasserstoffreste mit 1 bis 30 C-Atomen, vorzugsweise Kohlenwasserstoffreste mit 1 bis 2 C-Atomen oder Phenylreste, oder OR⁸-Reste darstellen,
g 0 bis 10, vorzugsweise 1 bis 3,
h 1 bis 11, vorzugsweise 1 bis 4,
I 0 bis 3, vorzugsweise < 2
R¹⁰ aliphatische oder aromatische Kohlenwasserstoffreste mit 1 bis 30 C-Atomen sind, die eine oder mehrere Amin- oder OH- oder SH-Gruppen tragen und gegebenenfalls verzweigt sind und gegebenenfalls durch Amin- oder Etherfunktionen unterbrochen oder OR⁸ oder Polyether sind.
R¹¹ aliphatische oder aromatische Kohlenwasserstoffreste mit 1 bis 30 C-Atomen sind, die gegebenenfalls verzweigt oder H sind.
k eine ganze Zahl zwischen 0 und 10,
j eine ganze Zahl zwischen 8 und 1.000 ist.

8. Verwendung der Verbindungen gemäß den Ansprüchen 1 bis 5 und 7 als Konditioniermittel in Haarbehandlungsmitteln und Haarnachbehandlungsmitteln.

9. Verwendung der Verbindungen gemäß den Ansprüchen 1 bis 5 und 7 als Konditioniermittel für Haarbehandlungsmittel und Haarnachbehandlungsmittel und zur Verbesserung der Haarstruktur.

10. Haarbehandlungsmittel und Haarnachbehandlungsmittel enthaltend 0,05 bis 10 Gew.-% mindestens einer der Verbindungen gemäß den Ansprüchen 1 bis 5 und 7, 0 bis 10 Gew.-% eines oder mehrerer Emulgatoren, 0 bis 10 Gew.-% eines oder mehrerer Konsistenzgeber, 0 bis 10 Gew.-% eines oder mehrerer vorzugsweise kationischer Tenside, 0 bis 20 Gew.-% eines oder mehrerer kosmetischer Öle oder Emollients sowie übliche Hilfs- und Zusatzstoffe in üblichen Konzentrationen, und zusätzlich enthaltend ein oder mehrere haarkosmetische Wirkstoffe ausgesucht aus der Gruppe der Proteinhydrolysate pflanzlichen oder tierischen Ursprungs auf Basis Keratin, Collagen, Elastin, Weizen, Reis, Soja, Milch, Seide, Mais oder Vitamine, Panthenol, Pyrrolidoncarbonsäure, Bisabolol, Pflanzenextrakte, Kreatin, Ceramide sowie UV-absorbierende Mittel.

11. Verwendung der Verbindungen gemäß den Ansprüchen 1 bis 5 und 7 zur Herstellung von glanzverbesserten Pflegeformulierungen.

12. Verwendung der Verbindungen gemäß den Ansprüchen 1 bis 5 und 7 in Trocknungshilfen für Autowaschstraßen.

## Claims

1. Polysiloxanes containing guanidino groups and of the general formula (I) 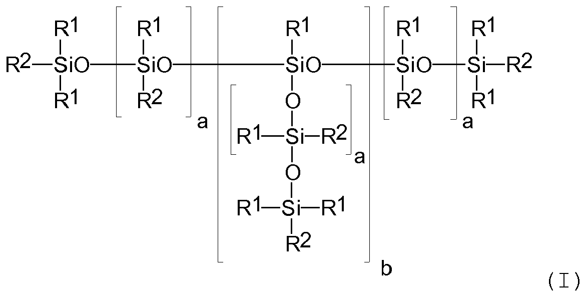 in which the radicals
R¹ are identical or different and are an optionally branched hydrocarbon radical which optionally contains double bonds and has 1 to 30 carbon atoms or phenyl radicals or -OR¹¹ or -OH,
R² can in part have the meaning of the radicals R¹ and the other radicals R², independently of one another, are radicals of the formulae (Ia, Ib or Ic)
R² = -M-G (Ia)
R² = -M-Q⁺ A⁻ (Ib)
R² = - (M)ₓ-S (Ic)
with the proviso that, in the average molecule, at least one radical R² is a radical of the formula -M-G (Ia), in which
G is a guanidino group with the general formula (Ia¹, Ia²) 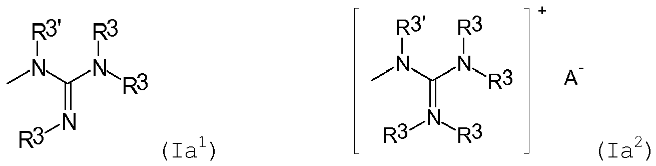 and/or salts or hydrates thereof, in which
R³ independently of the others, is hydrogen or an optionally branched hydrocarbon radical optionally comprising double bonds,
R³' may be R³ or an alkylene group which is joined to M via carbon atoms or heteroatoms and in so doing forms a 5-to 8-membered ring, and
M is a divalent or polyvalent hydrocarbon radical having at least 4 carbon atoms which has one hydroxyl group and which is interrupted by one or more oxygen atoms or nitrogen atoms or quaternary ammonium groups or ester or amide functions,
Q+ is a radical of the formula (Id) 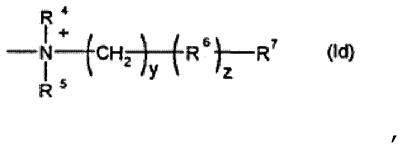
R4, R5 are alkyl radicals having 1 to 4 carbon atoms,
R⁶ is 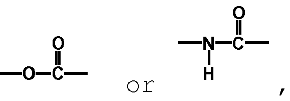
R⁷ may be a monovalent hydrocarbon radical having 1 to 22 carbon atoms,
y is 0 to 6
z is 0 or 1,
A⁻ is an inorganic or organic anion which originates from a customary physiologically compatible acid HA,
S is H, a polyalkylene oxide polyether of the general formula CₘH₂ₘO (C₂H₄O)ₙ (C₃H₆O)ₒR⁸ in which
m is 1 to 6, in particular 3, 6,
n,o independently of one another are 0 to 100, in particular 0 to 20 and the polyether has a molecular weight between 100 and 6000 g/mol and
R⁸ is H or an optionally branched aromatic or alicyclic hydrocarbon radical optionally containing double bonds and having 2 to 30 carbon atoms, preferably 4 to 22 carbon atoms, or a UV-absorbing group, in particular cinnamic acid or methoxycinnamic acid,
x is 0 or 1 and
a independently of the others has a value of from 8 to 1000, preferably 20 to 200, and
b has a value from 0 to 10.

2. Compounds according to Claim 1, **characterized in that** at least one of the radicals R² carries a guanidino group, and R¹ may be a methyl, ethyl, n-propyl, isopropyl, n-butyl, iso-butyl or phenyl radical.

3. Compounds according to Claim 1 and 2, **characterized in that** M is identical or different and is one of the radicals selected from the group

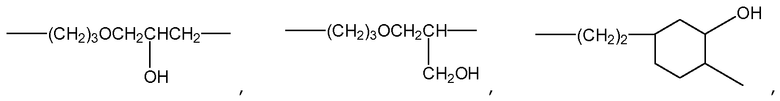

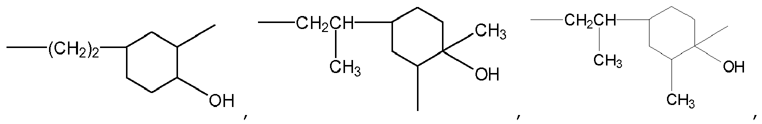

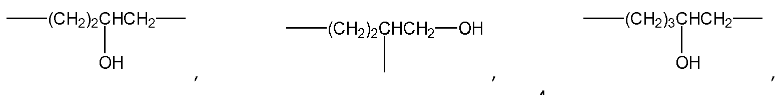

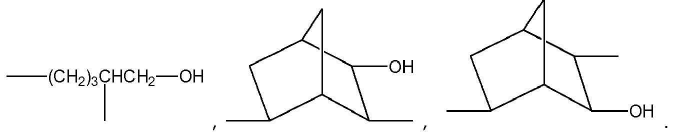

4. Compounds according to Claim 1, **characterized in that** A⁻, an inorganic or organic anion, originates from a customary physiologically compatible acid HA, such as carbonic acid, phosphoric acid, hydrochloric acid, sulfuric acid, and formic acid, acetic acid, propionic acid, heptanoic acid, caprylic acid, nonanoic acid, capric acid, undecanoic acid, lauric acid, myristic acid, palmitic acid, stearic acid, arachidic acid, behenic acid, cyclopentanecarboxylic acid, cyclohexanecarboxylic acid, acrylic acid, methacrylic acid, vinylacetic acid, crotonic acid, 2-/3-/4-pentenoic acid, 2-/3-/4-/5-hexenoic acid, lauroleic acid, myristoleic acid, palmitoleic acid, oleic acid, gadoleic acid, sorbic acid, linoleic acid, linolenic acid, pivalic acid, ethoxyacetic acid, phenylacetic acid, lactic acid, 2-ethylhexanoic acid, oxalic acid, glycolic acid, malic acid, malonic acid, succinic acid, tartaric acid, glutaric acid, citric acid, adipic acid, pimelic acid, suberic acid, azelaic acid, sebacic acid, benzoic acid, o-/m-/p-toluic acid, phenylacetic acid, salicylic acid, 3-/4-hydroxybenzoic acid, p-toluenesulfonic acid, benzoic acid, salicylic acid, cinnamic acid, 4-methoxycinnamic acid, 4-aminobenzoic acid, 4-bis(hydroxypropyl)aminobenzoic acid, 4-bis(poly-ethoxy)aminobenzoic acid, 4-dimethylaminobenzoic acid, 3-imidazol-4-ylacrylic acid, 2-phenylbenzimidazole-5-sulfonic acid, 3,3'-(1,4-phenylenedimethine)bis(7,7-dimethyl-2-oxo-bicyclo[2.2.1]heptane-1-methanesulfonic acid), 2-hydroxy-4-methoxybenzophenone-5-sulfonic acid and 3-(4'-sulfo)benzylidenebornan-2-one, phthalic acids or completely or partially hydrogenated derivatives thereof, such as hexahydro- or tetrahydrophthalic acid and mixtures thereof, in particular lactic acid, tartaric acid, acetic acid and hydrochloric acid.

5. Compounds according to Claims 1 to 4, **characterized in that** each Q is identical or different and is a cationic radical chosen from the group consisting of capryldimethylamine, lauryldimethylamine, cocodimethylamine, myristyldimethylamine, cetyldimethylamine, stearyldimethylamine, behenyldimethylamine, oleyldimethylamine, capryloylamidopropyldimethylamine, lauramidopropyldimethylamine, cocamidopropyldimethylamine, myristamidopropyldimethylamine, palmitamidopropyldimethylamine, stearamidopropyldimethylamine, behenamidopropyldimethylamine, oleamidopropyldimethylamine, undecylenamidopropyldimethylamine, ricinoleamidopropyldimethylamine, and guanidinopropyldimethylamine.

6. Method of producing the compounds according to Claim 1 to 5, **characterized in that** a siloxane containing at least one epoxy group is reacted at elevated temperature with guanidine compounds of the general formula
Z-G,
in which
G has the meaning given above and
Z is a hydrocarbon radical containing at least 2 carbon atoms, preferably 3 to 6, and optionally comprising heteroatoms, in particular N, which may be bonded to R³' via a carbon atom or heteroatom and in so doing forms a 5- to 8-membered ring,
and/or salts thereof by methods known per se.

7. Polysiloxanes containing guanidino groups which are obtained by reacting silanes containing guanidino groups and of the general formula II with one or more different silanes of the general formula III and one or more different OH- or OR¹¹-functional siloxanes of the general formula IV, optionally in the presence of a catalyst which promotes condensation and/or water and emulsifiers 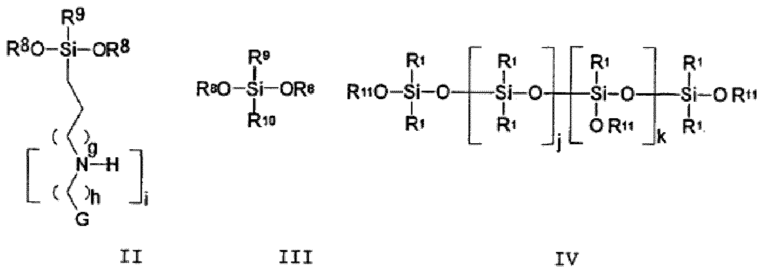 in which
R¹ are identical or different and are an optionally branched hydrocarbon radical which optionally contains double bonds and has 1 to 30 carbon atoms or phenyl radicals or -OR¹¹ or -OH,
R⁸ are identical or different and are hydrocarbon radicals having 1 to 30 carbon atoms, preferably hydrocarbon radicals having 1 to 2 carton atoms or phenyl radicals,
R⁹ are identical or different and are hydrocarbon radicals having 1 to 30 carbon atoms, preferably hydrocarbon radicals having 1 to 2 carbon atoms or phenyl radicals, or OR⁸ radicals,
g is 0 to 10, preferably 1 to 3,
h is 1 to 11, preferably 1 to 4,
i is 0 to 3, preferably < 2,
R¹⁰ are aliphatic or aromatic hydrocarbon radicals having 1 to 30 carbon atoms which carry one or more amine or OH or SH groups and are optionally branched and are optionally interrupted by amine or ether functions, or OR⁸ or polyether,
R¹¹ are aliphatic or aromatic hydrocarbon radicals having 1 to 30 carbon atoms which are optionally branched or H,
k is an integer between 0 and 10,
j is an integer between 8 and 1000.

8. Use of the compounds according to Claims 1 to 5 and 7 as conditioners in hair treatment compositions and hair aftertreatment compositions.

9. Use of the compounds according to Claims 1 to 5 and 7 as conditioners for hair treatment compositions and hair aftertreatment compositions and for improving the structure of the hair.

10. Hair treatment compositions and hair aftertreatment compositions comprising 0.05 to 10% by weight of at least one of the compounds according to Claims 1 to 5 and 7, 0 to 10% by weight of one or more emulsifiers, 0 to 10% by weight of one or more consistency regulators, 0 to 10% by weight of one or more preferably cationic surfactants, 0 to 20% by weight of one or more cosmetic oils or emollients, and customary auxiliaries and additives in customary concentrations, and additionally comprising one or more hair cosmetic active ingredients selected from the group of protein hydrolysates of vegetable or animal origin based on keratin, collagen, elastin, wheat, rice, soya, milk, silk, corn or vitamins, panthenol, pyrrolidonecarboxylic acid, bisabolol, plant extracts, creatine, ceramides, and UV-absorbing agents.

11. Use of the compounds according to Claims 1 to 5 and 7 for producing care formulations with improved shine.

12. Use of the compounds according to Claims 1 to 5 and 7 in drying auxiliaries for car washes.

## Revendications

1. Polysiloxanes contenant des groupes guanidino, de formule générale (I) 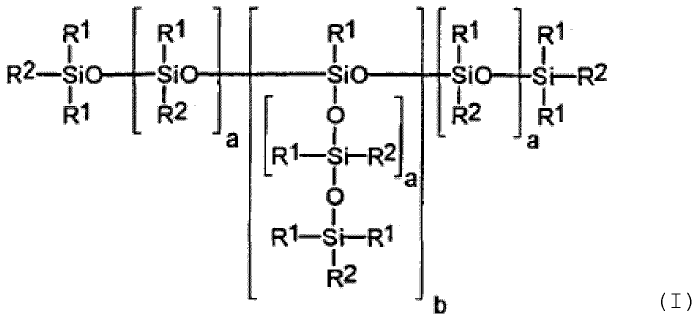 dans laquelle les radicaux
R¹ sont identiques ou différents et représentent un radical hydrocarboné éventuellement ramifié, comportant éventuellement des doubles liaisons, ayant de 1 à 30 atomes de carbone, ou des radicaux phényle ou -OR¹¹ ou -OH,
R² peuvent avoir pour une partie la signification des radicaux R ¹ et les autres radicaux R ² sont, indépendamment les uns des autres, des radicaux de formules (Ia, Ib ou Ic)
R² = -M-G (Ia)
R² = -M-Q⁺ A⁻ (Ib)
R ² = - (M) ₓ-S (Ic)
étant entendu que dans la molécule moyenne au moins un radical R² est un radical de formule -M-G (Ia),
formules dans lesquelles
G est un groupe guanidino de formule générale (Ia¹, Ia²) 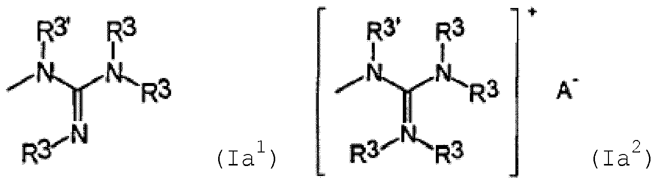 et/ou ses sels ou hydrates, où
R³ représente chaque fois indépendamment un atome d'hydrogène ou un radical hydrocarboné éventuellement ramifié, comportant éventuellement des doubles liaisons,
peut être R³ ou un groupe alkylène qui est relié à M par des atomes de carbone ou des hétéroatomes et forme ainsi un cycle à 5-8 chaînons et
M est un radical hydrocarboné di- ou plurivalent ayant au moins 4 atomes de carbone, qui comporte un groupe hydroxy et qui est interrompu par un ou plusieurs atomes d'oxygène ou atomes d'azote ou groupes ammonium quaternaires ou une ou plusieurs fonctions ester ou amide,
Q⁺ est un radical de formule (Id) 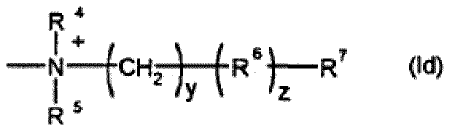
R⁴, R⁵ sont des radicaux alkyle ayant de 1 à 4 atomes de carbone,
R⁶ est 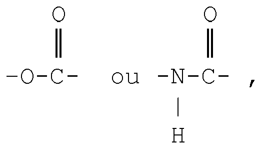
R⁷ peut être est un radical hydrocarboné monovalent ayant de 1 à 22 atomes de carbone,
y vaut de 0 à 6
z vaut 0 ou 1,
A⁻ est un anion organique ou inorganique qui provient d'un acide HA usuel, physiologiquement acceptable,
S est H, un polyoxyalkylène-polyéther de formule générale CₘH₂ₘO(C₂H₄O)ₙ (C₃H₆O)ₒR⁸ où
m vaut 1 à 6, en particulier 3, 6,
n, o valent, indépendamment l'un de l'autre, 0 à 100, en particulier 0 à 20, et le polyéther présente une masse moléculaire comprise entre 100 et 6 000 g/mole et
R⁸ est H ou un radical hydrocarboné aromatique ou alicyclique éventuellement ramifié, comportant éventuellement des doubles liaisons, ayant de 2 à 30 atomes de carbone, de préférence 4 à 22 atomes de carbone, ou un groupe absorbant les UV, en particulier l'acide cinnamique ou l'acide méthoxycinnamique,
x est 0 ou 1 et
a a chaque fois indépendamment une valeur de 8 à 1 000, de préférence de 20 à 200 et
b a une valeur de 0 à 10.

2. Composés selon la revendication 1, **caractérisés en ce qu'**au moins l'un des radicaux R² porte un groupe guanidino et R¹ peut être un radical méthyle, éthyle, n-propyle, isopropyle, n-butyle, isobutyle ou phényle.

3. Composés selon les revendications 1 et 2, **caractérisés en ce que** M, le même ou différent, est l'un des radicaux, choisi dans l'ensemble

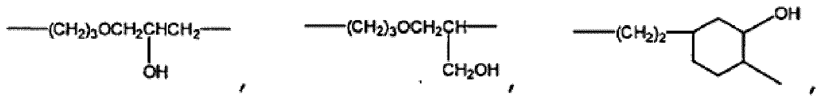

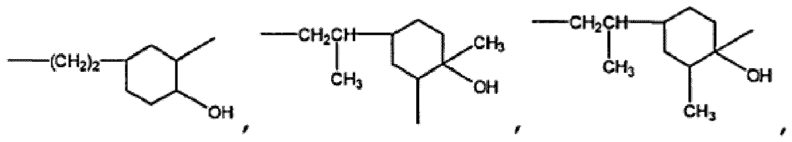

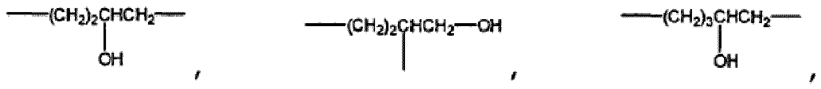

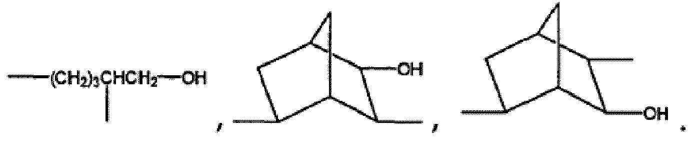

4. Composés selon la revendication 1, **caractérisés en ce que** A⁻, un anion inorganique ou organique, provient d'un acide HA usuel physiologiquement acceptable, tel que l'acide carbonique, l'acide phosphorique, l'acide chlorhydrique, l'acide sulfurique ainsi que l'acide formique, l'acide acétique, l'acide propionique, l'acide heptanoïque, l'acide caprylique, l'acide nonanoïque, l'acide caprique, l'acide undécanoïque, l'acide laurique, l'acide myristique, l'acide palmitique, l'acide stéarique, l'acide arachidique, l'acide béhénique, l'acide cyclopentane-carboxylique, l'acide cyclohexanecarboxylique, l'acide acrylique, l'acide méthacrylique, l'acide vinylacétique, l'acide crotonique, l'acide 2-/3-/4-penténoïque, l'acide 2-/3-/4-/5-hexénoïque, l'acide lauroléique, l'acide myristoléique, l'acide palmitoléique, l'acide oléique, l'acide gadoléique, l'acide sorbique, l'acide linoléique, l'acide linolénique, l'acide pivalique, l'acide éthoxyacétique, l'acide phénylacétique, l'acide lactique, l'acide 2-éthylhexanoïque, l'acide oxalique, l'acide glycolique, l'acide malique, l'acide malonique, l'acide succinique, l'acide D-tartrique, l'acide glutarique, l'acide citrique, l'acide adipique, l'acide pimélique, l'acide subérique, l'acide azélaïque, l'acide sébacique, l'acide benzoïque, l'acide o-/m-/p-toluylique, l'acide phénylacétique, l'acide salicylique, l'acide 3-/4-hydroxybenzoïque, l'acide p-toluènesulfonique, l'acide benzoïque, l'acide salicylique, l'acide cinnamique, l'acide 4-méthoxycinnamique, l'acide 4-aminobenzoïque, l'acide 4-bis (hydroxypropyl) aminobenzoïque, l'acide 4-bis(polyéthoxy)aminobenzoïque, l'acide 4-diméthyl-aminobenzoïque, l'acide 3-imidazol-4-yl-acrylique, l'acide 2-phénylbenzimidazole-5-sulfonique, l'acide 3,3'-(1,4-phénylènediméthine)-bis(7,7-diméthyl-2-oxobicyclo[2.2.1]heptane-1-méthanesulfonique), l'acide 2-hydroxy-4-méthoxybenzophénone-5-sulfonique et la 3-(4'-sulfo)benzylidènebornan-2-one, des acides phtaliques ou leurs dérivés partiellement ou totalement hydrogénés, tels que l'acide hexahydro- ou tétrahydrophtalique et des mélanges de ceux-ci, en particulier l'acide lactique, l'acide d-tartrique, l'acide acétique et l'acide chlorhydrique.

5. Composés selon les revendications 1 à 4, **caractérisés en ce que** Q, le même ou différent, est un radical cationique choisi dans l'ensemble constitué par la capryldiméthylamine, la lauryldiméthylamine, la cocodiméthylamine, la myristyldiméthylamine, la cétyldiméthylamine, la stearyldiméthylamine, la béhényldiméthylamine, l'oléyldiméthylamine, la capryloylamidopropyldiméthylamine, la lauramidopropyldiméthylamine, la cocosamidopropyldiméthylamine, la myristamidopropyldiméthylamine, la palmitamidopropyldiméthylamine, la stéaramidopropyldiméthylamine, la béhénamidopropyldiméthylamine, l'oléamidopropyldiméthylamine, l'undécylène-amidopropyldiméthylamine, la ricinolamidopropyldiméthylamine ainsi que la guanidinopropyldiméthylamine.

6. Procédé pour la préparation des composés selon l'une quelconque des revendications 1 à 5, **caractérisé en ce qu'**on fait réagir selon des procédés connus en soi un siloxane contenant au moins un groupe époxy, à température élevée, avec des composés de type guanidine de formule générale
Z-G,
dans laquelle
G a la signification indiquée plus haut et
Z est un radical hydrocarboné contenant au moins 2, de préférence 3 à 6, atomes de carbone, éventuellement contenant des hétéroatomes, en particulier N, qui peut être lié à R³' par un atome de carbone ou hétéroatome et forme ainsi un cycle à 5-8 chaînons,
et/ou leurs sels.

7. Polysiloxanes contenant des groupes guanidino, qui sont obtenus par la réaction de silanes contenant des groupes guanidino, de formule générale II avec un ou plusieurs silanes différents, de formule générale III, et un ou plusieurs siloxanes différents, à fonction OH ou OR¹¹, de formule générale IV, éventuellement en présence d'un catalyseur facilitant la condensation et/ou d'eau et d'émulsifiants 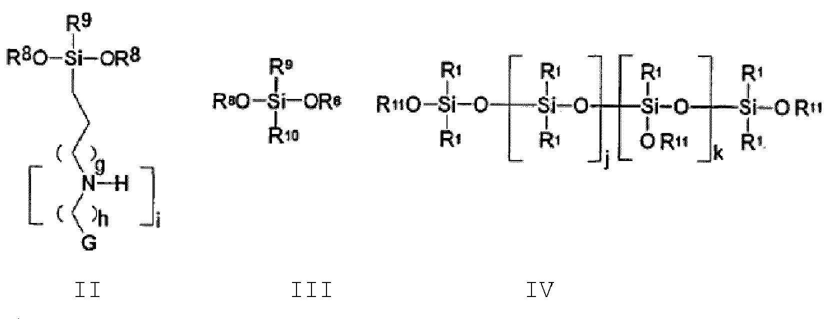 où
R¹ sont identiques ou différents et représentent un radical hydrocarboné éventuellement ramifié, comportant éventuellement des doubles liaisons, ayant de 1 à 30 atomes de carbone, ou des radicaux phényle ou -OR¹¹ ou -OH,
R⁸ sont identiques ou différents et représentent des radicaux hydrocarbonés ayant de 1 à 30 atomes de carbone, de préférence des radicaux hydrocarbonés à 1 ou 2 atomes de carbone ou des radicaux phényle,
R⁹ sont identiques ou différents et représentent des radicaux hydrocarbonés ayant de 1 à 30 atomes de carbone, de préférence des radicaux hydrocarbonés ayant 1 ou 2 atomes de carbone ou des radicaux phényle ou des radicaux OR⁸,
g vaut 0 à 10, de préférence 1 à 3,
h vaut 1 à 11, de préférence 1 à 4,
i vaut 0 à 3, de préférence < 2,
R¹⁰ sont des radicaux hydrocarbonés aliphatiques ou aromatiques, ayant de 1 à 30 atomes de carbone, qui portent un ou plusieurs groupes amino ou OH ou SH et éventuellement sont ramifiés et éventuellement sont interrompus par des fonctions amine ou éther, ou sont OR⁸ ou des polyéthers,
R¹¹ sont des radicaux hydrocarbonés aliphatiques ou aromatiques ayant de 1 à 30 atomes de carbone, qui sont éventuellement ramifiés, ou H,
k est un nombre entier compris entre 0 et 10,
j est un nombre entier compris entre 8 et 1 000.

8. Utilisation des composés selon les revendications 1 à 5 et 7, en tant que conditionneur dans des produits de traitement capillaire et produits d'après-traitement capillaire.

9. Utilisation des composés selon les revendications 1 à 5 et 7, en tant que conditionneur pour produits de traitement capillaire et produits d'après-traitement capillaire et destiné à l'amélioration de la structure du cheveu.

10. Produit de traitement capillaire et produit d'après-traitement capillaire, contenant 0,05 à 10 % en poids d'au moins l'un des composés selon les revendications 1 à 5 et 7, 0 à 10 % en poids d'un ou plusieurs émulsifiants, 0 à 10 % en poids d'un ou plusieurs agents de consistance, 0 à 10 % en poids d'un ou plusieurs tensioactifs de préférence cationiques, 0 à 20 % en poids d'une ou plusieurs huiles cosmétiques ou d'un plusieurs émollients cosmétiques ainsi que des adjuvants et additifs usuels aux concentrations usuelles, et en outre contenant une ou plusieurs substances actives cosmétiques capillaires choisies dans le groupe constitué par des hydrolysats de protéines d'origine animale ou végétale, à base de kératine, collagène, élastine, blé, riz, soja, lait, soie, maïs ou des vitamines, le panthénol, l'acide pyrrolidinecarboxylique, le bisabolol, des extraits végétaux, la créatine, des céramides ainsi que des absorbeurs UV.

11. Utilisation des composés selon les revendications 1 à 5 et 7, pour la fabrication de compositions de soin améliorant le brillant.

12. Utilisation des composés selon les revendications 1 à 5 et 7, en tant que produits auxiliaires de séchage pour stations de lavage d'automobiles.
